# EUROPEAN PATENT SPECIFICATION

(11) **EP 3 302 609 B1**
(45) Date of publication and mention of the grant of the patent: **09.10.2019**
(21) Application number: 16725647.8
(22) Date of filing: 29.04.2016
(51) Int. Cl.: A61M 1/16, B01J 20/02, B01J 20/06, B01J 20/34, B01J 20/28

(54) **RECHARGER FOR RECHARGING ZIRCONIUM PHOSPHATE AND ZIRCONIUM OXIDE MODULES**
NACHLADEGERÄT ZUM NACHLADEN VON ZIRKONPHOSPHAT- UND ZIRKONOXIDMODULEN
DISPOSITIF DE RECHARGE POUR RECHARGER DES MODULES DE PHOSPHATE DE ZIRCONIUM ET D'OXYDE DE ZIRCONIUM

(30) Priority: 26.05.2015 US 201514722119; 26.05.2015 US 201514722068
(43) Date of publication of application: 11.04.2018
(73) Proprietor: Medtronic, Inc., Minneapolis, Minnesota 55432 (US)
(72) Inventor: GERBER, Martin T., Maple Grove, MN 55369 (US); HOBOT, Christopher M., Rogers, MN 55374 (US); LURA, David B., Maple Grove, MN 55311 (US); MENON, Kanjimpuredathil Muralikrishna, Bangalore Karnataka 560037 (IN); PATIL, Kaustubh R., Bangalore Karnataka 560048 (IN); PARAMESWARAN, Mahesh, Bangalore Karnataka 560048 (IN); NARULA, Rohit Kumar, Uttarakhand 248001 (IN); JEYACHANDRAN, Ramkumar, Bangalore Karnataka 560066 (IN)
(74) Representative: Maschio, Antonio
(86) International application number: PCT/US2016/030304
(87) International publication number: WO 2016/191039

(56) References cited:
- WO-A1-2015/060914
- WO-A1-2015/199765
- WO-A1-2015/199863
- WO-A1-2015/199864
- US-A1- 2015 251 161
- US-A1- 2015 251 162

## Description

### FIELD OF THE INVENTION

The invention relates to rechargers for recharging sorbent materials within sorbent modules. The rechargers can recharge zirconium phosphate, zirconium oxide, or both zirconium phosphate and zirconium oxide and can be linked together to share resources and/or infrastructure

### BACKGROUND

Zirconium phosphate and zirconium oxide are used in sorbent dialysis to remove waste and unwanted solutes from spent dialysate. Generally, zirconium phosphate removes ammonium, potassium, calcium, and magnesium ions from dialysate while the zirconium oxide removes anions such as phosphate or fluoride ions. Both materials are usually packaged together in a cartridge of some type or packed in separate cartridges. Usually, sorbent cartridges are discarded and replaced after use. The discarded sorbent cartridges are broken down and the individual materials separated from each other. Because zirconium phosphate and zirconium oxide are expensive and rechargeable, sorbent reprocessers treat the recovered zirconium phosphate and zirconium oxide with chemical solutions. The recycling process requires transporting the materials to reprocessing facilities and involves laborious recycling steps in addition to recharging the sorbent materials. Further, the sorbent material cannot be immediately reused, and must be added to a new sorbent cartridge and repackaged for sale. Safe disposal of the chemical waste from solutions used to recharge the materials may also require additional steps such as neutralizing the recharging solutions. Conventional methods drive up costs and infrastructure requirements, and increase complexity and waste.

Hence, there is a need for systems and methods that can quickly and effectively recharge sorbent materials without the need to remove the spent sorbent materials from the sorbent cartridge or sorbent modules. There is further a need for systems and methods that can selectively recharge both zirconium phosphate and zirconium oxide either concurrently or independently. There is further a need for systems and methods that can quickly and effectively recharge different sorbent materials within a single recharging system. There is also a need for a system that can recharge both zirconium oxide and zirconium phosphate to allow for automatic neutralization of the recharging solutions allowing safe disposal without additional treatment. The need extends to dual cartridge systems where only one of cartridge is being recharged. There is a need for multiply connectable systems and related methods that can selectively recharge both zirconium phosphate and zirconium oxide for easy and quick use that can scale to use shared resources and/or infrastructure.

### SUMMARY OF THE INVENTION

The invention is defined by the appended claims.

The invention is drawn to a recharger for recharging zirconium oxide and/or zirconium phosphate. In a first aspect, the recharger can have a first receiving compartment for a first sorbent module; the first receiving compartment comprising a first sorbent module inlet and a first sorbent module outlet; one or more fluid sources fluidly connected to the first sorbent module inlet through a first set of one or more fluid connectors; one or more pumps positioned on the one or more fluid connectors for pumping fluid from the one or more fluid sources to the first sorbent module inlet; and a first effluent line fluidly connected to the first sorbent module outlet, the recharger comprising further a second receiving compartment for a second sorbent module; the second receiving compartment having a second sorbent module inlet and a second sorbent module outlet; wherein the one or more fluid sources are fluidly connected to the second module inlet through a second set of one or more fluid connectors; one or more pumps positioned on the one or more fluid connectors for pumping fluid from the one or more fluid sources to the second sorbent module inlet; and a second effluent line fluidly connected to the second sorbent module outlet, wherein the first effluent line and the second effluent line are fluidly connected to a common drain line; and wherein the drain line is fluidly connected to any one of a drain, a common reservoir, or combinations thereof.

In any embodiment, the sorbent module can be a zirconium phosphate module. In any embodiment, the one or more fluid sources can be any one of a water source, a disinfectant source, a brine source, and combinations thereof. The brine source can contain any one of a solution of sodium chloride, sodium acetate, acetic acid, and combinations thereof. In any embodiment, the concentration of sodium chloride can be between 2.5 M and 4.9 M, the concentration of sodium acetate between 0.3 M and 1.1 M, and the concentration of acetic acid between 0.2 M and 0.8 M.

In any embodiment, the sorbent module can be a zirconium oxide module.

In any embodiment, the fluid sources can include a water source, a disinfectant source, and a base source. In any embodiment, the base source can contain sodium hydroxide in a concentration of between 0.5 and 2.0 M.

In any embodiment, the first sorbent module can be a zirconium phosphate module; and the second sorbent module can be a zirconium oxide module. Alternatively, the first sorbent module can be a zirconium oxide module; and the second sorbent module can be a zirconium phosphate module. In any embodiment, either the first sorbent module or the second sorbent modules can be a zirconium phosphate module; or either the first sorbent module or the second sorbent modules can be a zirconium oxide module.

In any embodiment, the first and second sorbent modules can each be zirconium phosphate module or can each be zirconium oxide modules.

In any embodiment, the recharger can have multiple fluid sources.

In any embodiment, the recharger can have at least one module bypass line; wherein the module bypass line is positioned upstream of the first sorbent module inlet and is fluidly connected to the first effluent line.

In any embodiment, the recharger can have a second module bypass line; wherein the second module bypass line is positioned upstream of the second sorbent module inlet and is fluidly connected to the effluent line.

In any embodiment the drain line can have a static mixer.

In any embodiment, the first module inlet can be fluidly connectable to the first module outlet and/or second module inlet is fluidly connectable to the second module outlet.

In any embodiment, the fluid sources can be selected from the group of a water source, a base source, a disinfectant source, a brine source, and combinations thereof.

In any embodiment, at least one fluid source can be fluidly connected to a second set of one or more connectors in a second recharger.

In any embodiment, either or both of the sorbent module inlet and sorbent module outlet can be positioned on a flexible connector.

In a second aspect, the invention contemplates a dialysis system having one or more sorbent rechargers described in any embodiment wherein the one or more sorbent rechargers are fluidly connected to a common set of the one or more fluid sources. Any features of the first aspect may be included in the second aspect, and any feature of the second aspect can be included in the first aspect.

Any of the features disclosed as being part of the invention can be included in the invention, either alone or in combination.

### BRIEF DESCRIPTION OF THE DRAWINGS

FIG. 1 shows a recharger for recharging zirconium phosphate and zirconium oxide modules.
FIG. 2 shows a recharger fluidly connected to external fluid sources.
FIG. 3 shows multiple rechargers fluidly connected to a single set of fluid sources.
FIG. 4 shows a receiving compartment in a recharger.
FIG. 5A shows a recharging flow path for recharging zirconium phosphate and zirconium oxide.
FIG. 5B shows a recharging flow path for recharging zirconium phosphate and is an exploded left side of FIG. 5A.
FIG. 5C shows a recharging flow path for recharging zirconium oxide and is an exploded right side of FIG. 5A.
FIG. 6A shows a recharging flow path for recharging zirconium phosphate and zirconium oxide with in-line mixing of recharging solutions.
FIG. 6B shows a recharging flow path for recharging zirconium phosphate with in-line mixing of recharging solutions and is an exploded right side of FIG. 6A.
FIG. 6C shows a recharging flow path for recharging zirconium oxide with inline mixing of recharging solutions and is an exploded left side of FIG. 6A.
FIG. 7 shows a timeline for concurrent recharging of zirconium oxide and zirconium phosphate.
FIG. 8 shows a timeline for independent recharging of zirconium phosphate.
FIG. 9 shows a timeline for independent recharging of zirconium oxide.
FIG. 10 shows material layers in a module sorbent cartridge including reusable modules.
FIG. 11 shows multiple sorbent modules connected together to form a sorbent cartridge.

### DETAILED DESCRIPTION OF THE INVENTION

Unless defined otherwise, all technical and scientific terms used herein generally have the same meaning as commonly understood by one of ordinary skill in the relevant art.

The articles "a" and "an" are used herein to refer to one or to more than one (*i.e.,* to at least one) of the grammatical object of the article. By way of example, "an element" means one element or more than one element.

A "base source" is a fluid or concentrate source from which a basic solution can be obtained.

A "brine source" is a fluid or concentrate source from which a brine solution can be obtained. As used herein, a brine solution can refer to any solution comprising acids, bases and/or salts.

The term "comprising" includes, but is not limited to, whatever follows the word "comprising." Use of the term indicates the listed elements are required or mandatory but that other elements are optional and may be present.

A "common reservoir" can be a container for collecting fluid of any type from one or more fluid sources including fluid lines or other reservoirs. The "common reservoir" can for example, store used or waste fluids.

The term "common set" refers to sharing any grouping of components, modules, reservoirs, or fluid connectors. For example, a "common set of fluid sources" can refer to shared set of fluid sources that is used by one or more rechargers.

The term "consisting of' includes and is limited to whatever follows the phrase "consisting of." The phrase indicates the limited elements are required or mandatory and that no other elements may be present.

The term "consisting essentially of' includes whatever follows the term "consisting essentially of' and additional elements, structures, acts or features that do not affect the basic operation of the apparatus, structure or method described.

The terms "contain," "containing," or "contained" as used herein means to keep a material within a specific place. "Contain" can refer to materials placed within a component, absorbed onto a component, bound to a component, or any other method of keeping the material in a specific place.

A "disinfectant source" is a fluid or concentrate source from which a disinfectant solution can be obtained. The disinfectant solution can be an acidic solution, such as a peracetic acid solution, or any other solution capable of disinfecting reusable sorbent modules.

The term "downstream" refers to a position of a first component in a flow path relative to a second component wherein fluid will pass by the second component prior to the first component during normal operation. The first component can be said to be "downstream" of the second component, while the second component is "upstream" of the first component.

A "drain" is a fluid line through which fluids may be disposed.

A "drain line" is a fluid line through which used or waste fluid may flow for disposal. The drain line can be connected to a drain, or to a container or reservoir for later disposal of the fluid.

An "effluent line" is a fluid passageway, tube, or path of any kind into which fluid exiting a container, module, or component will flow.

A "flexible connector" is a connector that can be bent, twisted or otherwise deformed without substantial damage or fluid blockage.

A "fluid" is a liquid substance optionally having a combination of gas and liquid phases in the fluid. Notably, a liquid, as used herein, can therefore also have a mixture of gas and liquid phases of matter.

The term "fluidly connectable," "fluidly connect," "for fluid connection," and the like, refer to the ability of providing for the passage of fluid, gas, or a combination thereof, from one point to another point. The two points can be within or between any one or more of compartments, modules, systems, components, and rechargers, all of any type. The connection can optionally be disconnected and then reconnected.

A "fluid connector," "fluid connection," and the like describe a connection between two components wherein fluid, gas, or a combination thereof, can flow from one component, through a connector or a component for connection, to another component. The connector provides for a fluid connection in its broadest sense and can include any type of tubing, fluid or gas passageway, or conduit between any one or more components of the invention. The connection can optionally be disconnected and then reconnected.

The term "fluid line mixing" refers to mixing fluids at a location or junction wherein flow at the location of junction can, in part, mix one or more fluids.

A "fluid source" is a source from which a fluid or concentrate may be obtained.

The term "mixing" generally refers to causing one or more fluids from any source to combine together. For example, "mixing" can include laminar or turbulent flow at a location in a fluid line or a junction. Another example of "mixing" can include receiving one or more fluids in a component configured to receive fluids from one or multiple sources and to mix the fluids together in the component. Additionally, mixing can refer to the dissolution of a solid or solids with a fluid, wherein the solid or solids is dissolved in the fluid.

A "module bypass line" refers to a fluid line that provides for movement of fluid between two points without passing through a module.

A "module inlet" is a connector through which a fluid, slurry, or aqueous solution can enter a sorbent module.

A "module outlet" is a connector through which a fluid, slurry, or aqueous solution can exit a sorbent module.

The term "positioned" or "position" refers to a physical location of a component or structure.

The term "pump" refers to any device that causes the movement of fluids, gases, or combinations thereof, by applying suction or pressure.

The terms "pumping," "pumped," or to "pump" refers to moving a fluid, gas, or combination thereof, with a pump.

A "receiving compartment" is a space within a recharger into which a sorbent module to be recharged is placed.

A "sorbent recharger" is an apparatus designed to recharge at least one sorbent material.

"Recharging" refers to treating a sorbent material to restore the functional capacity of the sorbent material to put the sorbent material back into a condition for reuse or use in a new dialysis session. In some instances, the total mass, weight and/or amount of "rechargeable" sorbent materials remain the same. In some instances, the total mass, weight and/or amount of "rechargeable" sorbent materials change. Without being limited to any one theory of invention, the recharging process may involve exchanging ions bound to the sorbent material with different ions, which in some instances may increase or decrease the total mass of the system. However, the total amount of the sorbent material will in some instances be unchanged by the recharging process. Upon a sorbent material undergoing "recharging," the sorbent material can then be said to be "recharged." Recharging of rechargeable sorbent materials is not the same as replenishing of a sorbent material such as urease. Notably, urease is not "recharged," but can be replenished, as defined herein.

A "recharging flow path" is a path through which fluid can travel while recharging sorbent material in a reusable sorbent module.

The term "solution" refers to a fluid having a solute dissolved in water.

A "sorbent cartridge module" or "sorbent module" means a discreet component of a sorbent cartridge. Multiple sorbent cartridge modules can be fitted together to form a sorbent cartridge of two, three, or more sorbent cartridge modules. In some embodiments, a single sorbent cartridge module can contain all of the necessary materials for dialysis. In such cases, the sorbent cartridge module can be a "sorbent cartridge."

A "static mixer" is a component configured to receive fluids from one or multiple sources and to mix the fluids together. The static mixer may include components that agitate the fluids to further mixing.

The term "upstream" refers to a position of a first component in a flow path relative to a second component wherein fluid will pass by the first component prior to the second component during normal operation. The first component can be said to be "upstream" of the second component, while the second component is "downstream" of the first component.

A "valve" is a device capable of directing the flow of fluid, gas, or a combination thereof, by opening, closing or obstructing one or more pathways to control whether the fluid, gas, or combination thereof, is to travel in a path. One or more valves that accomplish a desired flow can be configured into a "valve assembly."

A "waste reservoir" is a container for collecting and storing used or waste fluids.

A "water source" is a fluid source from which water can be obtained.

A "zirconium oxide module" is a sorbent module containing zirconium oxide.

A "zirconium phosphate module" is a sorbent module containing zirconium phosphate.

### Zirconium Phosphate and Zirconium Oxide Rechargers

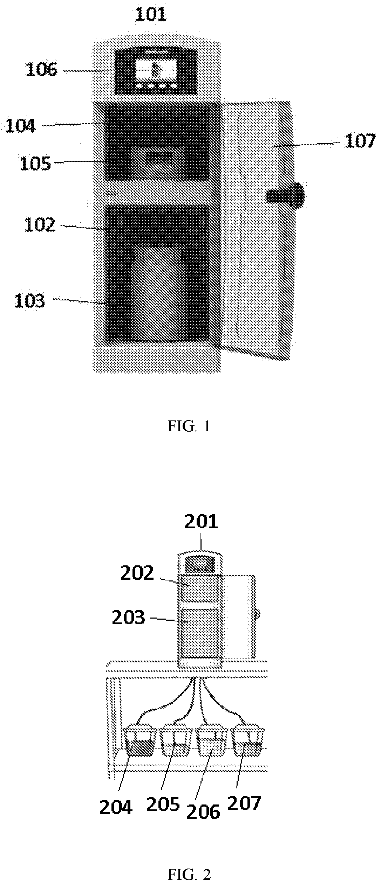

The disclosure relates to rechargers that can be used in recharging reusable sorbent modules such as reusable sorbent modules containing zirconium phosphate and/or zirconium oxide. The rechargers described can be used to recharge zirconium phosphate and zirconium oxide in reusable sorbent modules, either concurrently or independently. A recharger can be configured as shown in FIG. 1. The recharger **101** includes a receiving compartment **102** for receiving a reusable zirconium phosphate module **103.** Fluid connections (not shown in FIG. 1) connect to the top and bottom of the zirconium phosphate module **103** for passing recharging fluids into, through, and out of the reusable sorbent module **103.** As described, the recharging fluids replace ions bound to the sorbent materials during dialysis with new ions, recharging the zirconium phosphate within the zirconium phosphate module **103,** allowing reuse of the zirconium phosphate module **103** in dialysis. The recharger **101** also has a second receiving compartment **104** for receiving a reusable zirconium oxide module **105,** which is also fluidly connected to recharging fluid sources for recharging of the zirconium oxide module **105.** The recharger **101** can be configured to concurrently recharge a zirconium phosphate module **103** and a zirconium oxide module **105,** or to independently recharge either a zirconium phosphate module **103** or a zirconium oxide module **105.** A user interface **106** is provided to start or control the recharging process by the user. The user interface **106** can also provide the status of the recharging process to the user, such as the time to completion for each recharging step, or a time to complete the entire recharging process. User interface **106** provides alert messages if any problems are detected during recharging, such as leaks, occlusions, pump failures, or mismatched chemicals. A door **107** on the recharger **101** controls access to the receiving compartments **102** and **104** during operation.

In FIG. 1, the receiving compartments **102** and **104** may be of different sizes. Because less zirconium oxide is needed for dialysis than zirconium phosphate, the zirconium oxide module **105** is smaller than the zirconium phosphate module **103** and the receiving compartments **102** and **104** are sized accordingly. The zirconium phosphate receiving compartment **102** can be larger than the zirconium phosphate module **103** and the zirconium oxide receiving compartment **104** can be larger than the zirconium oxide module **105.** The larger space allows a user room to maneuver the fluid connectors and sorbent modules to connect the inlets and outlets on the sorbent modules to the inlets and outlets on the recharger **101.** Although shown as a recharger for recharging both zirconium phosphate and zirconium oxide in FIG. 1, one of skill in the art will understand that a recharger for recharging solely zirconium oxide or solely zirconium phosphate can be similarly constructed. A recharger for recharging a single sorbent material can have a single receiving compartment or multiple receiving compartments for receiving and recharging multiple modules containing the same sorbent material. Rechargers with any number of receiving compartments for recharging any number or combination of zirconium oxide and/or zirconium phosphate sorbent modules can be constructed. For example, a recharger with two zirconium phosphate receiving compartments and two zirconium oxide receiving compartments can be similarly constructed. The rechargers can have 1, 2, 3, 4, 5, 6, or more receiving compartments, each capable of receiving zirconium oxide or zirconium phosphate sorbent modules.

FIG. 2 illustrates a non-limiting embodiment of a recharger set up for recharging zirconium oxide and zirconium phosphate, either concurrently or independently. To recharge the sorbent materials, one or more recharging fluids can be passed through the reusable sorbent modules. As shown in FIG. 2, the recharger **201** can be fluidly connected to one or more recharging fluid sources, such as water source **204,** brine source **205,** disinfectant source **206,** and base source **207.** The recharger has a zirconium phosphate receiving compartment **202** and a zirconium oxide receiving compartment **203.** The recharger also has one or more pumps and valves (not shown in FIG. 2) for selectively delivering the recharging fluids from the fluid sources to the reusable modules. As shown in FIG. 2, the recharging fluid sources are housed external to the recharger **201.** Alternatively the recharging fluid sources can be housed within the recharger **201.** A drain line (not shown) is also connected to the recharger **201** for disposal of waste fluids exiting the reusable modules. The drain line is fluidly connected to a drain, or alternatively, the drain line can be fluidly connected to one or more waste reservoirs for storage and later disposal.

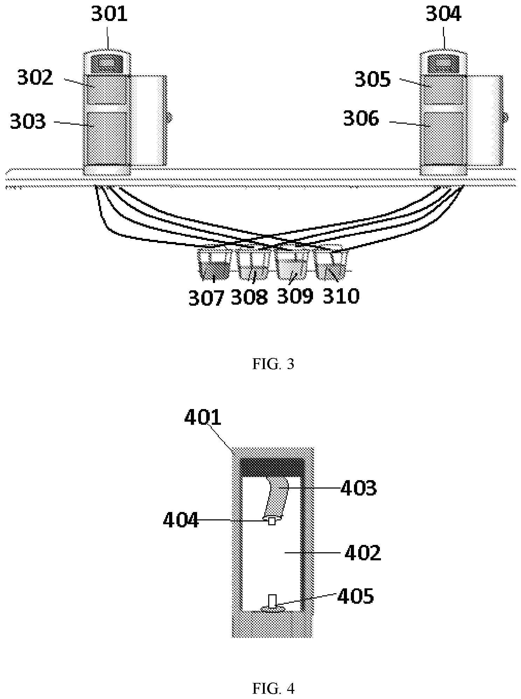

As illustrated in FIG. 3, multiple rechargers can be chained together and connected to a common set of fluid sources for sharing of infrastructure. A first recharger **301** having a zirconium phosphate receiving compartment **303** and zirconium oxide receiving compartment **302** is fluidly connected to water source **307,** brine source **308,** disinfectant source **309,** and base source **310.** A second recharger **304** having a zirconium oxide receiving compartment **305** and zirconium phosphate receiving compartment **306** is also fluidly connected to the same water source **307,** brine source **308,** disinfectant source **309,** and base source **310.** Any number of rechargers can be connected to the common set of fluid sources, including 2, 3, 4, 5, 6 or more rechargers, each fluidly connected to a single set of fluid sources and a single set of waste reservoirs. Connecting multiple rechargers to a single set of fluid sources can save space and materials and simplifies recharging multiple sets of reusable modules in a clinic or hospital setting. Each of the rechargers may include a separate drain line and/or separate waste reservoirs, or each recharger may be fluidly connected to a common drain line. The drain line can also be fluidly connected to any one of a drain, a common reservoir, or combinations thereof. Each of the connected rechargers can have separate heaters for heating the brine and/or disinfectant solutions, or centralized heaters can be included, with centralized heating of the shared solutions.

FIG. 4 illustrates a non-limiting embodiment of a sorbent module having a single receiving compartment **402** within a recharger **401.** The receiving compartment **402** includes a sorbent module inlet **404** on a fluid connector **403.** The fluid connector **403** is fluidly connected to the fluid sources, as described, allowing fluid from the fluid sources to enter into the reusable sorbent module. The receiving compartment also includes a sorbent module outlet **405** fluidly connected to an effluent line (not shown in FIG. 4). The effluent line can connect to a drain line, as described. Either or both of the sorbent module inlet **404** and sorbent module outlet **405** can be positioned on a flexible connector, such as fluid connector **403.** The flexible connectors allow for easier connection to a reusable sorbent module. Alternative embodiments of a recharger having multiple receiving compartments and flexible connectors are envisioned. The fluid connectors can be sized be of a sufficient length to allow direct connection of the sorbent module inlet **404** to the sorbent module outlet **405.** The direct connection between the sorbent module inlet **404** and sorbent module outlet **405** allows for recharging fluids to be passed through the fluid lines of the system even if a sorbent module is not being recharged. As described, when a single module, such as a zirconium oxide module, is being recharged, pumping the zirconium phosphate recharging fluids through the recharger and into a combined drain line allows for in-line neutralization of the zirconium oxide effluent. Alternatively, the same function can be accomplished by including a sorbent module bypass line in the recharging flow paths.

The rechargers can be used in any setting, including a clinic, at home, or in a mobile setting. In any setting, the rechargers can use a water tank or any other source of potable or deionized water. For use in a mobile setting, vans or trucks can carry the rechargers, the disinfectant source, the brine solution, the base solution, and optionally the water, to a location for recharging. For at home use, the brine solution, disinfectant solution, base solution, and optionally the water, may be prepackaged and shipped to a patient. The patient can connect each of the sources to the recharger to allow recharging and reuse of the sorbent modules in dialysis. As described, the rechargers can provide for inline mixing of chemicals, reducing the amount of chemicals required to be moved for use in a mobile setting. Inline mixing of chemicals allows for a smaller amount of concentrated solutions to be moved to a location in a mobile or at home setting, and water from a local water source, such as municipal drinking water, can be used to dilute the disinfectant, base, and/or brine inline. Alternatively, a deionized or purified water source can be provided in a mobile setting. Effluent from the sorbent modules can be collected and neutralized inline for immediate disposal in any drain, or can be collected for later neutralization and disposal offline. The ability to neutralize and dispose of the combined effluents in a drain allow for easier use in an at home or mobile setting, without the need for large waste reservoirs and further treatment.

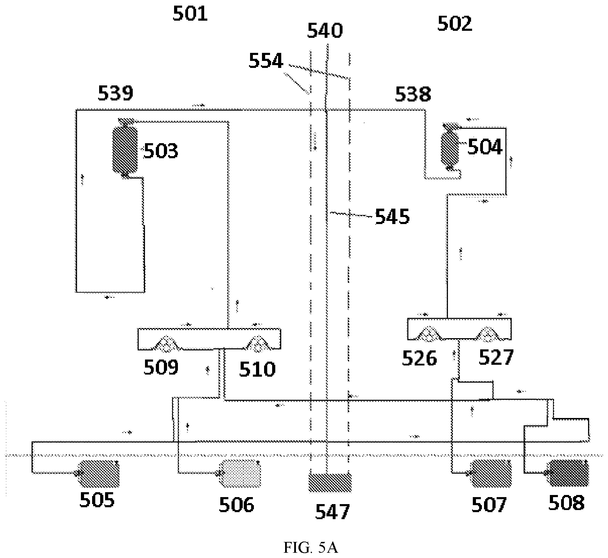

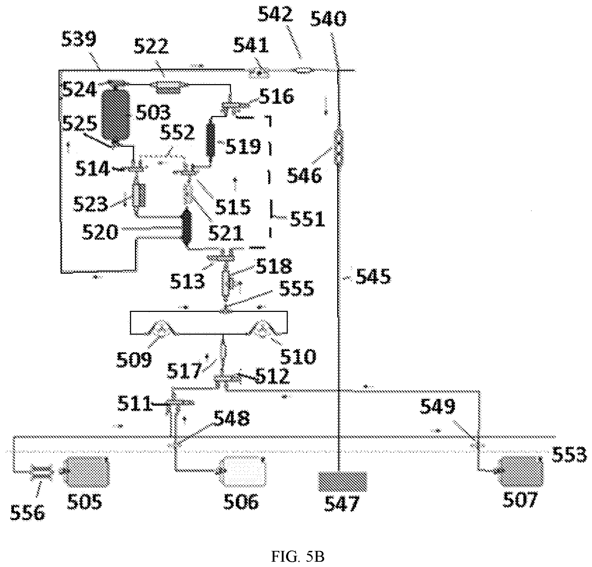

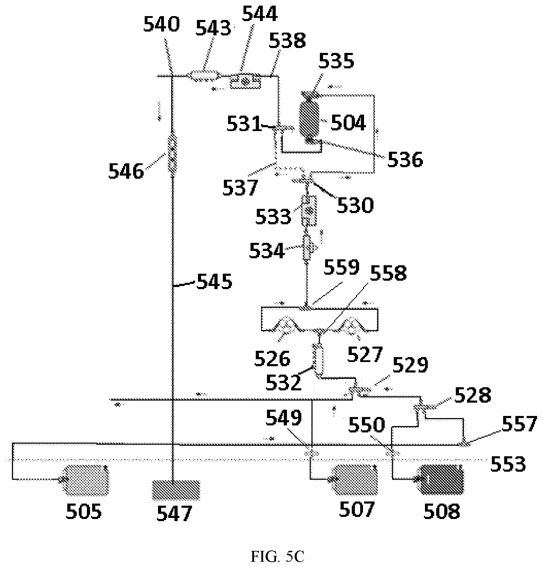

To recharge the sorbent materials, fluids from fluid sources are passed through the sorbent modules. The flow paths of the invention can be arranged as shown in FIG.'s 5A-C. FIG. 5A is a generalized view of a recharging flow path, with details shown in FIG.'s 5B and 5C. The recharging flow path can be divided into a zirconium phosphate recharging flow path **501** containing the zirconium phosphate module **503** and a zirconium oxide recharging flow path **502** containing zirconium oxide module **504.** Details of the zirconium phosphate recharging flow path **501 on** the zirconium phosphate side of line **554** are illustrated in FIG. 5B, while details of the zirconium oxide recharging flow path **502** on the zirconium oxide side of line **554** are illustrated in FIG. 5C. Although a dual cartridge recharger system is shown, single, two or more multiple cartridge recharger systems are envisioned. Any one of the recharger cartridge systems can be linked together to share resources for recharging the sorbent cartridge and can be adapted for large scale use. Similarly, the linked rechargers can be scaled down as demand for recharging decreases. The modular recharging set-up having more or less rechargers based on demand can be advantageously used where required.

In FIG. 5A, a zirconium phosphate recharging flow path **501** and a zirconium oxide recharging flow path **502** have a water source **505,** a brine source **506,** a disinfectant source **507,** and a base source **508.** The brine source **506,** disinfectant source **507,** and/or base source **508** can be a column containing a dry bed of the brine, acid, and/or base components. Alternatively, a powdered source of the brine, acid, and/or base components can be used. The dry bed or powdered source can be dissolved with an aqueous solution. A static mixer (not shown) can mix the single line coming through the column prior to entering the zirconium phosphate module **503** or zirconium oxide module **504.** Recharging the zirconium phosphate in a zirconium phosphate module **503** requires water, brine, and disinfectant. The water source **505,** the brine source **506,** and the disinfectant source **507** can be fluidly connected to the zirconium phosphate recharging flow path **501.** Similarly, recharging zirconium oxide module **504** in zirconium oxide recharging flow path **502** requires water, base, and disinfectant. The water source **505,** the disinfectant source **507,** and the base source **508** can be fluidly connected to the zirconium oxide recharging flow path **502.** The zirconium phosphate recharging flow path **501** and zirconium oxide recharging flow path **502** can be operated simultaneously or independently. Disinfectant source **507** can contain any type of disinfectant compatible with zirconium phosphate and zirconium oxide capable of disinfecting the reusable sorbent modules. In any embodiment, the acid source **507** can contain peracetic acid. In any embodiment, the peracetic acid can be a solution of between 0.5 % and 2% peracetic acid in water. The disinfectant source can alternatively contain any other disinfectant compatible with zirconium phosphate and zirconium oxide modules, including bleach or citric acid. The brine source **506** can have an acid, a base, and a sodium salt.

During zirconium phosphate recharging, potassium, calcium, magnesium, and ammonium ions bound to the zirconium phosphate must be replaced by hydrogen and sodium ions. The final ratio of hydrogen to sodium ions on the recharged zirconium phosphate can be determined by the pH, buffer capacity, and sodium concentration of the brine solution used in the recharging process. The brine source **506** can be a mixture of sodium chloride, sodium acetate, and acetic acid. In one non-limiting brine solution, the sodium chloride concentration can be between 2.5 M and 4.9 M, the sodium acetate concentration can be between 0.3 M and 1.1 M, and acetic acid concentration can be between 0.2 M and 0.8 M. The water source **505** can contain any type of water, including deionized water. To recharge the zirconium phosphate in the zirconium phosphate module **503,** the disinfectant from disinfectant source **507** can flow to the zirconium phosphate module **503** to disinfect the zirconium phosphate module **503.** Fluid from the disinfectant source **507** can flow to valve **512** in the zirconium phosphate recharging flow path **501.** Zirconium phosphate pumps **509** and **510** provide a driving force to pump the fluid through the zirconium phosphate recharging flow path **501.** Use of two or more separate pumps can reduce wear on the pumps. Correspondingly, smaller pumps can be used. The two or more pumps can provide in-line mixing and intermittent pumping so at any given time, a single pump can pump fluid through the zirconium phosphate recharging flow path **501.** The two pumps can be used simultaneously or independently. The two or more pumps can provide fluid line mixing of one or more separate fluid streams when used simultaneously. The two or more pumps can operate asynchronously but used concurrently. For example, a first pump can operate for a time and a second pump remain off, then the first pump shut off with the second pump turning on. Multiple pumps at various timed pumping stages are envisioned as described herein. One of skill in the art will understand that a single zirconium phosphate pump can also accomplish the described pump functions.

In FIG. 5B, zirconium phosphate pumps **509** and **510** can pump fluid from disinfectant source **507** through valve **512** and valve **513.** Fluid can be pumped through three-way junction **555** to valve **516** and into zirconium phosphate module **503** through zirconium phosphate module inlet **524.** The illustrated junctions combine the inlet chemicals or water pumped by the two pumps such that higher flow rates can be achieved. During filling, fluid inside zirconium phosphate module **503** can be forced through zirconium phosphate module outlet **525** and into zirconium phosphate module effluent line **539.** The disinfectant can be sequestered in the zirconium phosphate module **503** to ensure disinfection. Heater **519** upstream of the zirconium phosphate module **503** can heat the disinfectant because disinfection can become more efficient at elevated temperatures. After disinfection, zirconium phosphate module **503** can be rinsed using water from water source **505.** Zirconium phosphate pumps **509** and **510** can pump water from water source **505** through valves **511** and **512** to valve **513.** The water can then be pumped through valves **515** and **516** through the zirconium phosphate module **503** through zirconium phosphate module inlet **524,** out zirconium phosphate module outlet **525** and into zirconium phosphate module effluent line **539.** Water can be pumped through the zirconium phosphate module **503** until all of the disinfectant is removed.

Fluid from brine source **506** can be pumped through the zirconium phosphate module **503** to load the zirconium phosphate module **503** with the proper ratio of sodium and hydrogen ions. Zirconium phosphate pumps **509** and **510** can pump fluid from brine source **506** to valve **511.** The brine can follow the same pathway as the water through zirconium phosphate module **503** and into zirconium phosphate module effluent line **539.** Heater **519** upstream of the zirconium phosphate module **503** can heat brine because recharging can become more efficient at elevated temperatures. Heat exchanger **520** can lessen the load on heater **519.** One or more heat exchangers and one or more heaters can be used. The heat exchanger **520** can be fluidly connected to zirconium phosphate module effluent line **539** and to zirconium phosphate module inlet **524** upstream of heater **519.** The heated fluid exiting the zirconium phosphate module **503** in zirconium phosphate module effluent line **539** can heat the incoming brine solution in heat exchanger **520.** The heat exchanger **520** can have at least a first chamber and a second chamber. Fluid in the zirconium phosphate inlet lines can pass through the first chamber of the heat exchanger **520,** and fluid in the zirconium phosphate effluent line **539** can pass through the second chamber of the heat exchanger **520.** The increased temperature of the zirconium phosphate effluent in the second chamber can heat the fluid in the zirconium phosphate inlet lines in the first chamber. The zirconium phosphate module **503** can be rinsed again by pumping water through the zirconium phosphate module **503.** A static mixer (not shown) can be positioned upstream of the zirconium phosphate module **503** and mix the solutions prior to entering the zirconium phosphate module **503.**

Various sensors can be used in the zirconium phosphate module recharging flow path **501** to ensure proper concentrations and temperatures as shown in FIG. 5B. For example, conductivity sensor **517** can ensure that the incoming water contains no defined level of ions that may interfere with the recharging process, and that the brine solution and disinfectant solution are at a desired concentration. Conductivity sensor **517** can also ensure that sufficient rinsing has occurred to remove brine and disinfectant solution. Pressure sensor **518** can monitor pressure in the zirconium phosphate inlet lines to ensure there are no occlusions or leaks and that the inlet pressures are in an acceptable range. Temperature sensor **522** can ensure that the brine solution is at the proper temperature before entering zirconium phosphate module **503** and to control heater **519.** Temperature sensor **523** can be placed in zirconium phosphate module effluent line **539** to monitor the temperature of the effluent which can be controlled by heat exchanger **520** and heater **519.** A flow sensor **521** can monitor the flow rates of the fluids in the zirconium phosphate recharging flow path **501** and control zirconium phosphate pumps **509** and **510.** One of skill in the art will understand that alternative arrangements of sensors can be used in FIG. 5B and that one or more additional sensors can be added. Further, the sensors can be placed at any appropriate position in the zirconium phosphate recharging flow path **501** to determine fluid parameters at various locations throughout the zirconium phosphate recharging flow path **501.**

Zirconium phosphate module bypass line **552** in FIG. 5B fluidly connects valve **515** to valve **514** in the zirconium phosphate effluent line **539.** Valves **515** and **516** can be controlled to direct fluid through the zirconium phosphate module bypass line **552** and into zirconium phosphate effluent line **539.** The dual flow path aspect of the recharging flow path depicted in FIG. 5A can neutralize the effluent from both the zirconium phosphate module **503** and zirconium oxide module **504** by mixing the acidic effluent from the zirconium phosphate module **503** with the basic effluent from zirconium oxide module **504.** If only zirconium oxide module **504** is being recharged using the flow path of FIG. 5C, the zirconium phosphate module bypass line **552** in FIG. 5B can be utilized to direct fluid from the brine source **506** to the zirconium phosphate effluent line **539** to neutralize the zirconium oxide effluent without the need to simultaneously recharge a zirconium phosphate module **503.** Alternatively, zirconium phosphate module inlet **524** can directly connect to zirconium phosphate module outlet **525.** The zirconium phosphate recharging flow path **501** can include a rinse loop **551** to fluidly connect valve **513** upstream of the heater **519** and heat exchanger **520** to valve **516,** bypassing heater **519** and heat exchanger **520.** The rinse loop **551** can rinse brine solution from the zirconium phosphate module **503.** By bypassing heater **519** and heat exchanger **520** through rinse loop **551,** the zirconium phosphate module **503** can be cooled faster.

In FIG. 5C, the zirconium oxide module **504** can be recharged by pumping disinfectant from disinfectant source **507** to the zirconium oxide module **504** to disinfect the zirconium oxide module **504.** Fluid from the disinfectant source **507** can be pumped to valve **529** in the zirconium oxide recharging flow path **502.** Zirconium oxide pumps **526** and **527** can pump fluid through the zirconium oxide recharging flow path **502.** As described, a single zirconium oxide pump is contemplated as an alternative to the dual pump system in FIG. 5C. Also, more than two zirconium oxide pumps are contemplated. The two or more zirconium oxide pumps can provide fluid line mixing of one or more separate fluid streams when used simultaneously. The two or more pumps can be asynchronous but used concurrently. For example, a first pump can operate for a time and a second pump remain off, then the first pump shut off with the second pump turning on. Multiple pumps at various timed pumping stages are envisioned as described herein. Zirconium oxide pumps **526** and **527** of FIG. 5C pump fluid from disinfectant source **507** through valve **529** to valve **530.** The fluid can flow to the zirconium oxide module **504** through zirconium oxide module inlet **535.** During filling, fluid inside zirconium oxide module **504** can flow through zirconium oxide module outlet **536** and into zirconium oxide module effluent line **538.** The disinfectant can be sequestered in zirconium oxide module **504** to ensure disinfection. The zirconium oxide module **504** can then be flushed with water from water source **505** after disinfection is completed. Zirconium oxide pumps **526** and **527** can pump water from water source **505** through valves **528** and **529** and junction **557** to valve **530.** The fluid passes through junctions **558** and **559** to reach valve **530.** The water can then be pumped to zirconium oxide module **504** through zirconium oxide module inlet **535** and out zirconium oxide module outlet **536** and into zirconium oxide module effluent line **538.** The zirconium oxide module **504** can be flushed with any volume of water required to ensure that the disinfectant is completely removed.

In FIG. 5C, zirconium oxide pumps **526** and **527** can pump fluid from base source **508** through valve **528** to zirconium oxide module **504.** The base source **508** can contain hydroxide ions to recharge zirconium oxide module **504.** The hydroxide ions can flow through zirconium oxide module **504** and into zirconium oxide module effluent line **538.** The base source **508** can be any suitable basic solution capable of replacing phosphate and other anions bound to the zirconium oxide with hydroxide ions. The hydroxide base can be any suitable base such as sodium hydroxide. One non-limiting example is sodium hydroxide having a concentration between 0.5M and 2.0M. Another non-limiting example is sodium hydroxide having a concentration at 90% or greater than 2% of the concentration of the recharging solution. A final rinse of the zirconium oxide module **504** can be performed by pumping water through the zirconium oxide recharging flow path **502** of FIG. 5A and zirconium oxide module **504.** Zirconium oxide recharging flow path **502** can also have a zirconium oxide module bypass line **537** fluidly connecting valve **530** in the zirconium oxide inlet line to valve **531** in the zirconium oxide effluent line **538** as shown in FIG. 5C Valves **530** and **531** can direct fluid through the zirconium oxide module bypass line **537** and into zirconium oxide effluent line **538.** Zirconium oxide module bypass line **537** can convey fluid directly from the base source **508** to the zirconium oxide effluent line **538** to neutralize the zirconium phosphate effluent without the need to simultaneously recharge a zirconium oxide module **504.** Alternatively, zirconium oxide module inlet **535** can be fluidly connected to zirconium oxide module outlet **536.** Multiple sensors can be included in the zirconium oxide recharging flow path **502** to monitor fluid concentration. For example, conductivity sensor **532** can monitor concentrations of the zirconium oxide recharging fluid; pressure sensor **534** can monitor pressure in the zirconium oxide inlet line and to detect leaks or occlusions. Flow sensor **533** can determine the flow rate of the fluid through the zirconium oxide inlet line and be used to control zirconium oxide pumps **526** and **527.** A static mixer (not shown) can be positioned upstream of the zirconium oxide module **504** and mix solutions prior to entering the zirconium oxide module **504.** A heater and heat exchanger (not shown) can be positioned in the zirconium oxide recharging flow path **502** to heat fluids prior to entering zirconium oxide module **504.** Heating fluid in the zirconium oxide recharging flow path **502** can reduce recharging times and allow disinfection with a base solution, such as sodium hydroxide. Heating the fluid also allows for reduced disinfection time with an acid source. A zirconium oxide rinse loop (not shown) can also be included to bypass the heater and heat exchanger during flushing.

Effluent from zirconium phosphate recharging flow path **501** can neutralize, either completely or in part, the effluent from zirconium oxide recharging flow path **502,** and vice versa. Zirconium phosphate effluent line **539** can be fluidly connected to zirconium oxide effluent line **538** at an effluent line junction **540** joining drain line **545,** which fluidly connects to drain **547.** Static mixer **546** can be used at or downstream of the effluent line junction **540** to mix zirconium phosphate effluent with zirconium oxide effluent.

Zirconium phosphate effluent line **539** of FIG. 5B and zirconium oxide effluent line **538** of FIG. 5C can be connected to a common reservoir for storage and disposal of the combined effluent. The common reservoir can receive and collect the zirconium phosphate and zirconium oxide effluents together. The collected effluents can be drained after appropriate volumes of each effluent have been added to achieve neutralization. A common reservoir can allow for neutralization of the zirconium phosphate and zirconium oxide effluents without synchronizing the recharging processes. A single common reservoir can be sized to support multiple recharge stations. Alternatively, the two fluid streams may be mixed through fluid line mixing at the effluent line junction **540.** Flow sensor **541** and conductivity sensor **542** can be placed in zirconium phosphate effluent line **539** to measure the flow rate and composition of the zirconium phosphate effluent as shown in FIG. 5B. Similarly, flow sensor **544** and conductivity sensor **543** can be positioned in the zirconium oxide effluent line **538** to measure the flow rate and composition of the zirconium oxide effluent of FIG. 5C. Data from flow sensors **541** and **544** and conductivity sensors **542** and **543** can determine if the combined effluent in drain line **545** is safe for disposal into a drain. One non-limiting example of safe is an effluent having a pH 5-9. Either zirconium phosphate effluent line **539** or zirconium oxide effluent line **538** can be connected simultaneously or independently to a waste reservoir (not shown) for disposal. Additional pH or conductivity sensors can be positioned downstream of the static mixer **546** to monitor and ensure safe disposal. Drain line **545** can also be connected to a common waste reservoir for storage and disposal of effluent. The common reservoir receives and collects the zirconium phosphate and zirconium oxide effluents together. The collected effluents can be drained after appropriate volumes of each effluent have been added to achieve neutralization. A common waste reservoir advantageously allows for neutralization of the zirconium phosphate and zirconium oxide effluents without synchronizing the recharging processes. Static mixer **546** can be unnecessary when a common reservoir is used.

Brine source **506,** disinfectant source **507,** and base source **508** can have filter **548,** filter **549,** and filter **550,** respectively to remove particulate matter. The one or more filters can remove particulate matter before fluid enters the zirconium oxide recharging flow path **502** or zirconium phosphate recharging flow path **501.** Water source **505** can have microbial filter **556** to remove microbes from the water before entering the flow paths. In FIG. 5C, the dashed line **553** represents a recharger housing. The fluid sources can be external to the recharger housing and fluidly connected to the lines located inside of the recharger housing. Alternatively, the fluid sources described can instead be housed within the recharger.

During recharging, fluid can be passed through the zirconium phosphate module **503** and/or the zirconium oxide module **504** opposite to a flow direction used during dialysis. For example, zirconium phosphate module inlet **524** can be used as the zirconium phosphate module outlet during dialysis, and zirconium phosphate module outlet **525** can be used as the zirconium phosphate module inlet during dialysis in FIG. 5B. Similarly, zirconium oxide module inlet **535** in FIG. 5C can be used as the zirconium phosphate module outlet during dialysis, and zirconium oxide module outlet **536** can be used as the zirconium phosphate module inlet during dialysis. Pumping the recharging fluid through the modules in the opposite direction relative to dialysis can improve the efficiency of the recharging process.

The zirconium phosphate recharging flow path **501** or zirconium oxide recharging flow path **502** of FIG. 5A can independently recharge zirconium phosphate or zirconium oxide. For example, a single flow path fluidly connecting zirconium phosphate module **503** of FIG. 5B via valve **512** and valve **513** to each of the water source **505,** brine source **506,** and disinfectant source **507** can independently recharge the zirconium phosphate module **503.** Similarly, a single flow path fluidly connecting zirconium oxide module **504** of FIG. 5C via valve **528** and valve **529** to each of the water source **505,** disinfectant source **507,** and base source **508** can independently recharge the zirconium oxide module **504.**

The water source **505,** brine source **506,** disinfectant source **507,** and base source **508** can recharge one or more reusable sorbent module of various sizes. The amount of water, brine, disinfectant, and base depends on the concentration of each of the recharging solutions, the size of the reusable sorbent modules, the amount of cations/anions removed, and the flow rate used to pass the solutions through the reusable modules. The amount of brine solution required can depend on the temperature to which the brine solution is heated. For example, a brine solution having between 2.5 M and 4.9 M sodium chloride, between 0.3 M and 1.1 M sodium acetate, and between 0.2 M and 0.8 M acetic acid at between 70°C and 90°C requires between 4.2-6.2 L of brine to recharge a zirconium phosphate module containing between 2 kg and 3.2 kg of zirconium phosphate loaded with 2 to 3 moles of ammonium, calcium, magnesium and potassium. The brine solution should have a volume of at least between 4.2 and 6.2 L and delivered at a flow rate of between 100 and 300 mL/min. A single brine source can be connected to multiple rechargers, or can recharge multiple zirconium phosphate modules in a single recharger. The brine source can have a significantly larger volume from 1-100X or greater to ensure that the brine source need not be refilled each time a zirconium phosphate is recharged. For a zirconium oxide module having between 220 and 340 g of zirconium oxide loaded with 200 mmols of phosphate, a base source having between 0.5 and 2.0 M sodium hydroxide and a flow rate between 30 and 150 mL/min requires between 1 and 4 L of base. The base source can be at least between 1 and 4 L in volume. For recharging multiple zirconium oxide modules, a larger base source can be used.

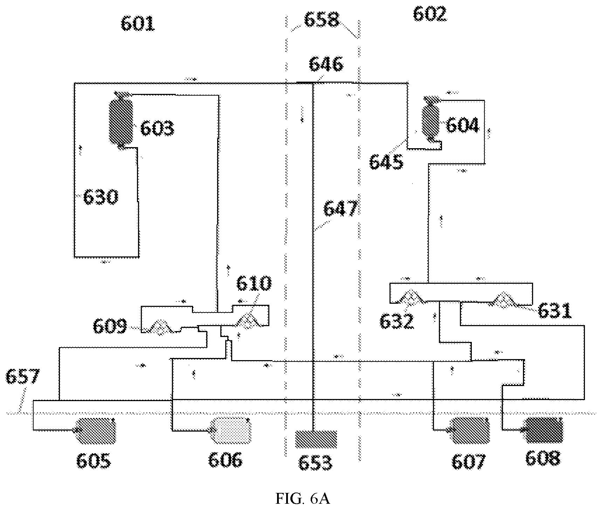

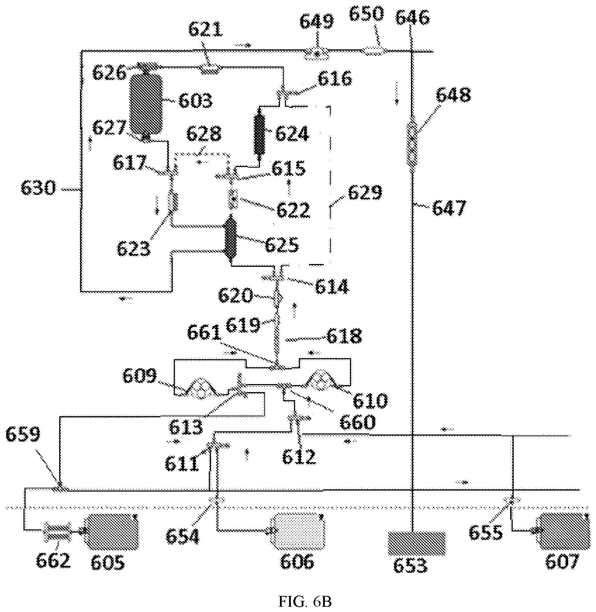

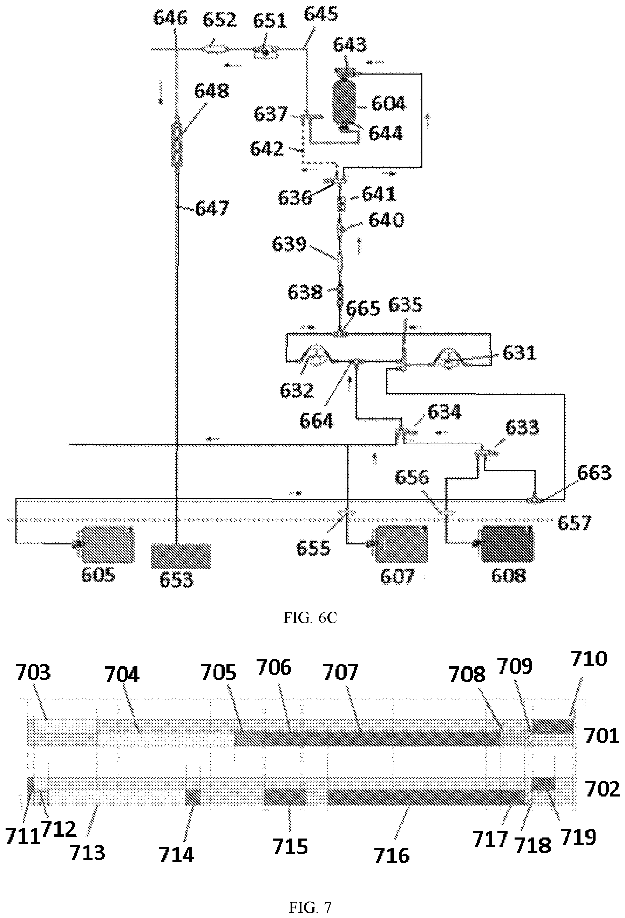

FIG. 6A is a generalized view of a recharging flow path having a zirconium phosphate recharging flow path **601** containing a zirconium phosphate module **603** and a zirconium oxide recharging flow path **602** containing a zirconium oxide module **604** with in-line mixing of recharging solutions. FIG. 6B illustrates a detailed view of zirconium phosphate recharging flow path **601** on the zirconium phosphate side of line **658,** and FIG. 6C illustrates a detailed view of zirconium oxide recharging flow path **602** on the zirconium oxide side of line **658.** The valves, pumps and static mixers illustrated in FIG.'s 6B and 6C allow for inline mixing of the recharging fluids. In FIG. 6A, the zirconium phosphate recharging flow path **601** and/or zirconium oxide recharging flow path **602** can be simultaneously or independently connected to a water source **605,** a brine source **606,** a disinfectant source **607,** and a base source **608.** Because recharging of the zirconium phosphate in a zirconium phosphate module **603** can require water, brine, and disinfectant, and because recharging of zirconium oxide in zirconium oxide module **604** can also require water, base, and disinfectant, the water source, **605,** the brine source **606,** and the disinfectant source **607** can be jointly connected to the zirconium phosphate recharging flow path **601,** and the water source **605,** the disinfectant source **607,** and the base source **608** can be jointly connected to the zirconium oxide recharging flow path **602.**

In FIG. 6A, zirconium phosphate recharging flow path **601** and zirconium oxide recharging flow path **602** can mix chemicals in-line to create the recharging solutions. Any of disinfectant source **607,** brine source **606,** and base source **608** can contain solutions having concentrations over the concentration of the components to be used in recharging the reusable modules. Water source **605** can dilute the disinfectant, brine, and base from the fluid sources prior to recharging. In FIG. 6B, zirconium phosphate pump **610** can pump disinfectant into the zirconium phosphate module **603** with in-line mixing of concentrated disinfectant from disinfectant source **607** from valve **612** through junctions **660** and **661** and into static mixer **618.** Concurrently, zirconium phosphate pump **609** can pump water through junction **659** and valve **613** and into static mixer **618** from water source **605.** Alternatively, the concentrated disinfectant and water can be mixed through fluid line mixing at the junction of the two fluid lines as shown in line junction **646** of FIG. 6A. The zirconium phosphate pumps **609** and **610** can pump a disinfectant solution having a specified concentration and composition to disinfect the zirconium phosphate module **603** via valves **612** and **613** of FIG. 6B. The disinfectant solution can flow from static mixer **618** through valve **614** to valve **616** and then into the zirconium phosphate module **603** through zirconium phosphate module inlet **626.** Fluid can exit zirconium phosphate module **603** through zirconium phosphate module outlet **627** into zirconium phosphate effluent line **630.** After disinfection of zirconium oxide module **603,** zirconium phosphate pumps **609** and **610** can pump water from water source **605** into zirconium phosphate module **603.** For example, zirconium phosphate pump **609** can pump water through valve **613** to zirconium phosphate module **603** while zirconium phosphate pump **610** can pump water through valves **611** and **612** to zirconium phosphate module **603.** Alternatively, zirconium phosphate pump **609** can pump water through valves **611, 612,** and **613** while zirconium phosphate pump **610** pumps water through valves **611** and **612.** During recharging, zirconium phosphate pumps **609** and **610** can pump brine through valve **611** to valve **612** from brine source **606** into static mixer **618.** If a concentrated brine solution is being used, zirconium phosphate pumps **609** and/or **610** can pump water from water source **605** to static mixer **618** to dilute the brine solution and generate a brine solution having a proper solute concentration for recharging the zirconium phosphate. After pumping brine through the zirconium phosphate module **603,** zirconium phosphate pump **609** can pump water through valves **611, 612** and **613** while zirconium phosphate pump **610** can pump water through valve **611** and **612.**

The zirconium phosphate recharging flow path **601** of FIG. 6B can have a heater **624** and heat exchanger **625.** One or more heat exchangers and one or more heaters can be used. The brine solution can be heated by the heater **624** upstream of the zirconium phosphate module **603.** Heat exchanger **625** can utilize the heat from brine exiting the zirconium phosphate module **603** to heat the incoming brine solution upstream of heater **624** to reduce the burden on heater **624.** As described, the zirconium phosphate recharging flow path **601** can also have an optional zirconium phosphate module bypass line **628** fluidly connecting valve **615** in the zirconium phosphate inlet line to valve **617** in the zirconium phosphate effluent line **630.** The zirconium phosphate module bypass line **628** can neutralize the zirconium oxide effluent with brine even if the zirconium phosphate module **603** is not being recharged. Zirconium phosphate recharging flow path **601** can have a rinse loop **629** connecting valve **614** upstream of the heater **624** and heat exchanger **625** to valve **616** to bypass heater **624** and heat exchanger **625** to rinse brine out of the zirconium phosphate module **603.**

Various sensors can be included in the zirconium phosphate recharging flow path **601** to ensure fluid parameters are within acceptable ranges. In FIG. 6B, conductivity sensor **619** can be placed downstream of static mixer **618** to ensure mixing and specified recharging fluid concentrations. Pressure sensor **620** can measure the fluid pressure and to identify leaks or occlusions. Flow sensor **622** can determine the flow rate of the fluid entering the zirconium phosphate module **603** and be used to control zirconium phosphate pumps **609** and **610.** Temperature sensor **621** can determine if the recharging fluid is a proper temperature range upon entering zirconium phosphate module **603** and relay data to a processor (not shown) that can control heater **624.** Temperature sensor **623** can determine the temperature of the zirconium phosphate effluent prior to entering heat exchanger **625.** Other sensor arrangements, including any number of conductivity, pressure, flow, and temperature sensors can be used.

In FIG. 6C, zirconium oxide pump **632** can pump disinfectant from disinfectant source **607** through valve **634** and into static mixer **638** to disinfect the zirconium oxide module **604** in zirconium oxide recharging flow path **602.** Zirconium oxide pump **631** can pump water from water source **605** through valve **635** to static mixer **638** to dilute the disinfectant from disinfectant source **607** to provide in-line mixing of the disinfectant solution. The diluted disinfectant can then be pumped through valve **636** to zirconium oxide module inlet **643** and into zirconium oxide module **604.** Effluent from the zirconium oxide module **604** can exit through zirconium oxide module outlet **644** and into zirconium oxide effluent line **645.** After disinfection, the disinfectant can be rinsed from the zirconium oxide module **604** by pumping water from water source **605** through valve **635** to zirconium oxide module **604** by zirconium oxide pump **631** while zirconium oxide pump **632** pumps water through valves **633** and **634** to zirconium oxide module **604.** Alternatively, zirconium oxide pump **631** can pump water through valves **633, 634,** and **631,** while zirconium oxide pump **632** pumps water through valves **633** and **634..** To recharge zirconium oxide module **604,** zirconium oxide pump **632** can pump base from base source **608** through valves **633** and **634** through junctions **664** and **665** to static mixer **638.** Water from water source **605** can be pumped by zirconium oxide pump **631** through junctions **663** and **665** into static mixer **638** to dilute the base by in-line mixing. Alternatively, the water and base can be mixed through fluid line mixing at the junction of the two fluid lines. Alternatively, the base can be pre-set using specified amounts of base in pre-packaged packets or containers. Diluted base can flow through the zirconium oxide recharging flow path **602** and through zirconium oxide module **604.** The zirconium oxide module **604** can be rinsed any numbers of times, as needed, by introducing water from water source **605** to the zirconium oxide module **604.** The zirconium oxide recharging flow path **602** can also have a zirconium oxide module bypass line **642** that fluidly connects valve **636** to valve **637** in the zirconium oxide effluent line **645** to bypass zirconium oxide module **604.** In this way, zirconium phosphate effluent can be neutralized with a base solution even if the zirconium oxide module **604** is not being recharged. A heater and heat exchanger (not shown) can be positioned in the zirconium oxide recharging flow path **602** to heat fluids prior to entering zirconium oxide module **604.** A zirconium oxide rinse loop (not shown) can also be included to bypass the heater and heat exchanger. Similarly, the zirconium oxide recharging flow path **602** can also have sensors for measurement and control over the recharging process. In FIG. 6C, a conductivity sensor **639** can be placed downstream of static mixer **638** to ensure that diluted recharging solutions have a desired concentration. Pressure sensor **640** can detect the pressure in the zirconium oxide recharging flow path **602** to detect leaks or occlusions. Flow sensor **641** can detect the flow rate of fluid in the zirconium oxide recharging flow path **602** and can be used to control zirconium oxide pumps **631** and **632.**

As shown in FIG. 6A, the present invention can provide in-line neutralization of the effluent from each of the zirconium phosphate recharging flow path **601** and zirconium oxide recharging flow path **602.** The zirconium phosphate effluent line **630** can be fluidly connected to zirconium oxide effluent line **645** at effluent line junction **646** and fluidly connected to drain line **647.** As shown in FIG.'s 6B and 6C, a static mixer **648** can be positioned at or downstream of the effluent line junction **646** to ensure mixing of the effluents from the zirconium phosphate recharging flow path **601** and zirconium oxide recharging flow path **602.** The combined effluent can be passed through the drain line **647** to drain **653,** or to a common waste reservoir (not shown), or to separate waste reservoirs. A conductivity sensor **650** as shown in FIG. 6B in zirconium phosphate effluent line **630** and a conductivity sensor **652** as shown in FIG. 6C in zirconium oxide effluent line **645** can determine the composition of the effluents. Flow sensor **649** in zirconium phosphate effluent line **630** of FIG. 6B and flow sensor **651** in zirconium oxide effluent line **645** of FIG. 6C can be used simultaneously or independently to measure the flow rates of each of the effluents. Determining the composition of the effluent fluids as well as the respective flow rates using one or more sensors described can monitor the system function and ensure that the combined effluent in drain line **647** is safe for disposal or storage.

Brine source **606,** disinfectant source **607,** and base source **608** can have filter **654,** filter **655,** and filter **656,** respectively to remove particulate matter prior to entering zirconium phosphate recharging flow path **601** or zirconium oxide recharging flow path **602.** The filters can also act as inline mixers to mix the solutions. Water source **605** can have microbial filter **662** to remove microbes from the water. Brine source **606,** disinfectant source **607,** and base source **608** can be housed outside of a recharger housing denoted by line **657.** The brine solution, disinfectant solution, and base solution can be generated through in-line mixing as described. Alternatively, pre-mixed solutions, concentrates, or infusates can be introduced into brine source **606,** disinfectant source **607,** and base source **608** and delivered to zirconium phosphate recharging flow path **601** or zirconium oxide recharging flow path **602.** For example, the brine solution in brine source **606** can be pre-mixed or provide in pre-packaged amounts in the proper concentrations and introduced into brine source **606,** disinfectant source **607,** and base source **608.**

In-line mixing can provide higher concentrations of solutes, lower fluid volumes required by the system, and physically smaller fluid reservoirs. The fluids can have suitable concentrations for use in the zirconium phosphate recharging flow path **601** or zirconium oxide recharging flow path **602.** For example, an initially high source of peracetic acid can be used in a concentration of between 20% and 40%. The zirconium phosphate recharging flow path **601** of FIG. 6B can dilute the peracetic acid or other disinfectant source by a factor of 20:1 to 40:1 to generate an disinfectant solution having a concentration between 0.5 % and 2%. The initial disinfectant concentration can be any concentration greater than 1%. Similarly, the base solution can be sodium hydroxide having an initial concentration between 14M and 22M. The zirconium oxide recharging flow path **602** of FIG. 6C can dilute the base solution by 18:1 to 22:1 to generate a base solution having a concentration between 0.8 and 1.0 M. The initial base solution concentration can be any concentration greater than or equal to 0.5 M. The brine solution can also be diluted in-line to generate a brine solution having a proper recharging concentration. The brine source **606** of FIG. 6A can be one or more reservoirs. For example, an acetic acid source, a sodium acetate source and a sodium chloride source can each be connected in place of single brine source **606.** Alternatively, an acetic acid source, a base source, and a sodium chloride source can be connected in place of the single brine source **606** with mixing of the base and acetic acid to generate the sodium acetate. The individual components can be added to the zirconium phosphate recharging flow path **601** in the proper ratios to generate the recharging brine.

The chemicals used in the recharging process can be packaged and shipped in any form. The chemicals can be packaged and shipped as solutions, either in proper concentrations for use in recharging or with higher concentrations for use in in-line mixing. In any embodiment, the chemicals may be packaged and shipped in pure form, such as 100% acetic acid or solid sodium chloride, sodium acetate, or sodium hydroxide.

FIG. 7 illustrates a non-limiting example of a timeline that can be used for concurrent or separate recharging of zirconium phosphate and zirconium oxide. Timeline **701** shows recharging zirconium phosphate and timeline **702** shows recharging zirconium oxide. As illustrated in timeline **701,** the zirconium phosphate recharging process can begin by introducing a disinfectant, such as peracetic acid, into the zirconium phosphate module, shown as step **703.** The time necessary to fill the zirconium phosphate module with the disinfectant can depend on the flow rate of the disinfectant solution and the volume of the zirconium phosphate module. The disinfectant can be delivered to the zirconium phosphate module in step **703** at a flow rate of between 200 and 500 mL/min, which can fill a zirconium phosphate module in a time of between 5-10 minutes. After filling the zirconium phosphate with the disinfectant solution, the disinfectant solution can be held in the zirconium phosphate module to ensure disinfecting of the zirconium phosphate module in step **704.** In any embodiment, the disinfectant can be held in the zirconium phosphate module for any length of time sufficient to disinfect the zirconium phosphate module, including between 5 and 20 minutes. Longer or shorter flushing times can be used depending on the need. The temperature of the disinfectant can be determined with a temperature sensor, and the hold time adjusted as necessary. For example, if the disinfectant temperature is 22°C, the hold time can be 5 minutes. The disinfectant can also be heated to minimize the hold time by heating the disinfectant to room temperature. During the hold time, the disinfectant flow can be stopped or reduced to a low flow condition, such as 5 to 75 ml/min. Holding the disinfectant, such as peracetic acid, in the module can build up pressure in the module, requiring periodic venting. To maintain the volume after venting, during which some fluid may leak, the disinfectant can be pumped into the module at a low flow rate during the venting. Alternatively, during the hold time, the disinfectant flow rate can be set to between 5 and 75 ml/min to prevent pressure buildup while maintaining fluid volume in the modules. The disinfectant solution can then be flushed from the zirconium phosphate module in step **705** by pumping water through the zirconium phosphate module. The water can flow through the zirconium phosphate module at a specified rate. A higher flow rate of the water in step **705** will cause a quicker flush time. The water can be pumped through the zirconium phosphate module at a rate of between 100 and 500 mL/min. Depending on the size of the zirconium phosphate module, the zirconium phosphate module can be flushed in about 5-10 minutes. As described, the system can utilize one or more sensors, such as pH sensors or conductivity sensors in the zirconium phosphate effluent lines to determine if disinfectant is fully flushed in step **705.** After flushing the disinfectant from the zirconium phosphate module in step **705,** brine solution can be pumped through the zirconium phosphate module to recharge the zirconium phosphate module starting in step **706.** The brine solution can be pumped through the zirconium phosphate module in step **706** at any rate. One of skill in the art will understand that a higher flow rate of brine solution may decrease the time necessary to recharge the zirconium phosphate, but may also decrease the efficiency of the process, resulting in the need for additional brine. Conductivity or pH sensors can determine if the zirconium phosphate module has been fully filled with brine.

The brine flow rate can be set to any flow rate, including between 150 and 250 mL/min. Depending on the size of the zirconium phosphate module, between 5 and 10 minutes may be needed for brine to reach the sensors in the zirconium phosphate effluent line. Once brine has reached the sensors in the effluent line, the brine can flow through the zirconium phosphate module in step **707** until recharging is complete. Recharging time can vary based on the flow rate of the brine solution, the concentration of the brine solution, and the temperature of the brine solution. For example, the brine solution can be heated during the recharging process between 65°C and 95°C. Recharging of zirconium phosphate can be more efficient at elevated temperatures. Conductivity sensors can be used to determine if step **708** has been completed by detecting the conducting of the fluid in the zirconium phosphate effluent line. If the conductivity of the effluent matches the conductivity of the brine, then no additional ions from the brine are being exchanged onto the zirconium phosphate, and recharging is complete. For example, steps **708, 709,** and **710** represent brine solution being flushed from the zirconium phosphate module with water. Flushing can continue through step **710** until the conductivity sensors in the zirconium phosphate effluent line determine no additional brine is being removed from the zirconium phosphate module.

As depicted in timeline **702,** zirconium oxide can be recharged concurrently or independently of zirconium phosphate. In step **711,** zirconium oxide recharging begins by rinsing the zirconium oxide module with water. The water rinse can flush leftover dialysate bicarbonate or any sodium hydroxide from the flow loop, which may react violently with acid necessary for disinfection. After flushing the zirconium oxide module with water in step **711,** disinfectant solution can be delivered to disinfect the module in step **712.** The time necessary to fill the zirconium oxide module with disinfectant depends on the size of the zirconium oxide module and the flow rate of the disinfectant. Because less zirconium oxide is needed for dialysis than zirconium phosphate, the zirconium oxide module may be smaller than the zirconium phosphate module, and therefore fill faster in step **712** as compared to the zirconium phosphate module in step **703.** Upon filling, the disinfectant can be sequestered in the zirconium oxide module to allow for disinfection in step **713.** The disinfectant can be held in the zirconium oxide module for any length of time, including between 5 and 20 minutes. The temperature of the disinfectant can be determined with a temperature sensor, and a hold time adjusted as necessary. For example, if the disinfectant temperature is 22°C, the hold time can be 5 minutes. The disinfectant can also be heated to minimize the necessary hold time. Upon disinfection, the disinfectant can be flushed from the zirconium oxide module in step **714.**

In step **715** the base solution flows through the zirconium oxide module to recharge the zirconium oxide. Step **715** continues until a basic solution is detected in the zirconium oxide effluent line. During simultaneous recharging, the basic effluent from the zirconium oxide recharging flow path neutralizes the acidic effluent from the zirconium phosphate recharging flow path. Once a basic effluent is detected in step **715,** the zirconium oxide recharging process can be halted until the acid brine is detected in the effluent of the zirconium phosphate module in step **706,** which may occur later due to size differences of the zirconium phosphate and zirconium oxide modules. After the acidic effluent is detected in the zirconium phosphate module, shown as step **706,** the base can continue to flow through the zirconium oxide module in step **716.** The flow rate of the base solution in step **716** can be any suitable rate. For example, the flow rate of the base solution can be between 30 and 150 mL/min. To ensure neutralization, the flow rate of the base in step **716** can depend on the flow rate of the brine in step **707.** As described, the base and effluent are each brought to a point equidistant to a junction between the zirconium phosphate and zirconium oxide effluent lines. Based on the conductivity of each effluent, the pumping is restarted at a ratio of speed that is needed for neutralization. The ratio could be 1:1 or any other ratio. Although described as using a conductivity sensor, the system can alternatively use a pH sensor or a combination of pH and conductivity sensors. A neutralization ratio can be calculated based on the relative pH, buffer capacity, and concentration of the zirconium phosphate effluent and zirconium oxide effluent. For example, a neutralization ratio of 1.5:1 means that 1.5 liters of the zirconium phosphate effluent will be required to fully neutralize one liter of zirconium oxide effluent. The flow rate of the base in step **716** can be set to half the flow rate of the brine solution, allowing full neutralization of both solutions. For example, the flow rate of the base in step **716** can be between 75 and 125 mL/min if the neutralization ratio is 1.5:1 and the brine flow rate is between 150 and 250 mL/min.

After the brine solution is detected in the effluent of the zirconium phosphate and the flushing of the brine begins in step **708,** the base solution can pass through the zirconium oxide module, shown as step **717** until the brine is mostly or fully flushed from the zirconium phosphate module, shown as step **709.** At this point, the base solution can be flushed from the zirconium oxide module, shown as step **718.** After confirming that the base has been flushed from the zirconium oxide module, flushing is completed in step **719.**

One of skill in the art will understand that the times and flow rates described in FIG. 7 can be altered within the scope of the invention. Higher flow rates can cause faster recharging of the modules. Times can be decreased by using more concentrated solutions, but may decrease efficiency. Specified concentrations, flow rates, and times can be set per the needs of the user, taking into account the cost of chemicals and need for fast recharging. The times and flow rates shown in zirconium oxide recharging timeline **702** can also be altered to reduce idle time. For example, the flow rate of the base solution in step **715** can be slowed down to reduce the time gap between steps **715** and **716.** If a single sorbent module is being recharged independently, or if a common reservoir is used for the zirconium phosphate and zirconium oxide recharging flow paths either inside or outside of the recharger,, the times and flow rates shown in FIG. 7 can be adjusted. Synchronizing the zirconium phosphate timeline **701** with the zirconium oxide timeline **702** is unnecessary because effluent is no longer neutralized in-line.

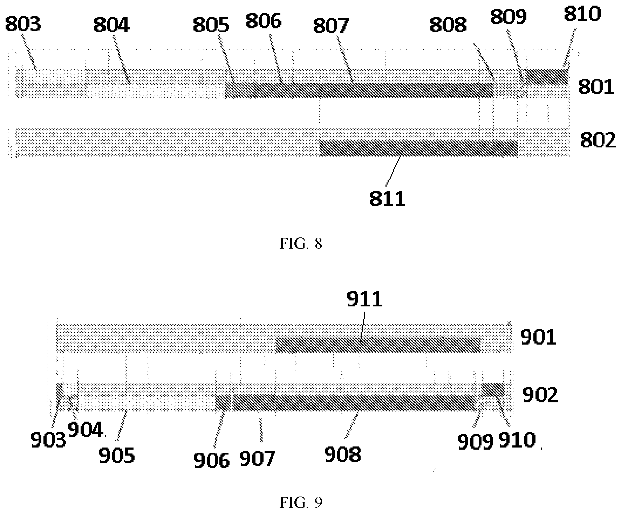

FIG. 8 illustrates a non-limiting example of a timeline that can be used for independent recharging of zirconium phosphate using the dual recharging flow path described herein. Timeline **801** shows recharging zirconium phosphate and timeline **802** shows the process for in-line neutralization without recharging a zirconium oxide module. As illustrated in timeline **801,** the zirconium phosphate recharging process can begin by introducing a disinfectant, such as peracetic acid, into the zirconium phosphate module, shown as step **803.** After filling the zirconium oxide with the disinfectant solution, the disinfectant solution can be sequestered in the zirconium oxide module to ensure disinfecting of the zirconium phosphate module in step **804.** The disinfectant solution can then be flushed from the zirconium phosphate module in step **805** by pumping water through the zirconium phosphate module at a specified rate. As described, the system can utilize one or more sensors, such as pH sensors or conductivity sensors in the zirconium phosphate effluent lines to determine if disinfectant is fully flushed in step **805.** After flushing the disinfectant from the zirconium phosphate module in step **805,** brine solution can be pumped through the zirconium phosphate module to recharge the zirconium phosphate module starting in step **806.** Once brine has reached the sensors in the effluent line, the brine can flow through the zirconium phosphate module in step **807** until recharging is complete. At the same time, a base solution can be pumped through the zirconium oxide recharging flow path in step **811** to neutralize the brine solution.

As described, conductivity sensors can be used to determine if step **808** has been completed by detecting the conducting of the fluid in the zirconium phosphate effluent line. If the conductivity of the effluent matches the conductivity of the brine, then no additional ions from the brine are being exchanged onto the zirconium phosphate, and recharging is complete. For example, steps **808, 809,** and **810** represent brine solution being flushed from the zirconium phosphate module with water. Flushing can continue through step **810** until the conductivity sensors in the zirconium phosphate effluent line determine no additional brine is being removed from the zirconium phosphate module. Once the conductivity sensors determine that the pH of the zirconium phosphate effluent is safe for disposal without additional treatment, the base solution in the zirconium oxide recharging flow path is stopped. The fluid flow rates and concentrations used in the process illustrated in FIG. 8 can be the same as the fluid flow rates and concentrations described with reference to FIG. 7.

FIG. 9 shows a timeline for independently recharging zirconium oxide. Timeline **902** shows the recharging of zirconium oxide and timeline **901** shows using the zirconium phosphate recharging flow path for in-line neutralization of the zirconium oxide effluent. In step **903,** zirconium oxide recharging begins by rinsing the zirconium oxide module with water to flush leftover dialysate bicarbonate, which may react violently with acid necessary for disinfection. After flushing the zirconium oxide module with water in step **903,** disinfectant solution can be delivered to disinfect the module in step **904.** Upon filling, the disinfectant can be sequestered in the zirconium oxide module to allow for disinfection in step **905.** Upon disinfection, the disinfectant can be flushed from the zirconium oxide module in step **906.**

In step **907** the base solution flows through the zirconium oxide module to recharge the zirconium oxide. Step **907** continues until a basic solution is detected in the zirconium oxide effluent line. Once the basic solution is detected in the zirconium oxide effluent line, brine is pumped through the zirconium phosphate recharging flow path for in-line neutralization of the basic zirconium oxide effluent in step **911.** The base solution continues to flow through the zirconium oxide module until recharging is complete in step **908.** After recharging the zirconium oxide in step **908,** the basic solution can be flushed in steps **909** and **910.** Conductivity sensors in the zirconium oxide effluent line determine when the basic solution is fully flushed, at which point the brine solution in step **911** can be stopped. The process illustrated in FIG. 9 can use the same flow rates and concentrations as described with respect to FIG. 7.

The zirconium oxide and zirconium phosphate sorbent modules can be recharged and reused any number of times. Alternatively, the sorbent modules may have a defined useful life, including a maximum number of recharge and reuse cycles. When a sorbent module reaches the end of the sorbent module's useful life, the sorbent module can be recycled or disposed of. A disinfection only cycle can disinfect the sorbent modules for safe disposal and/or recycling at the end of the sorbent module's useful life. In a disinfection only cycle, the disinfectant can be pumped into the sorbent module as described but the other recharge solutions would not be used. After disinfection, and optionally rinsing of the sorbent module, the sorbent module can be disposed or recycled safely.

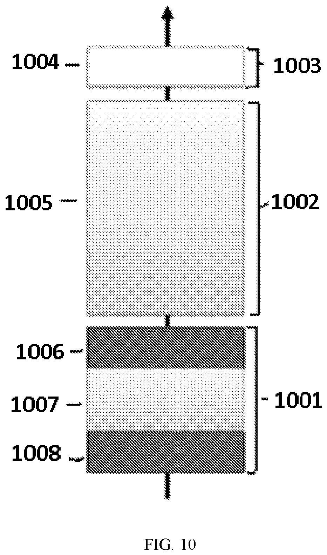

A non-limiting embodiment of a reusable sorbent cartridge having modules that can be separated and recharged by systems and methods of the present invention is shown in FIG. 10. The sorbent cartridge can be separated into reusable modules to facilitate recharging of one or more sorbent materials. In FIG. 10, the sorbent cartridge has a first sorbent module **1001,** a second sorbent module **1002,** and a third sorbent module **1003.** The first module **1001** can have a layer of activated carbon **1008,** a layer of alumina and urease **1007,** and a second layer of activated carbon **1006.** The activated carbon can remove many non-ionic solutes from the dialysate. The urease catalyzes the conversion of urea in the dialysate into ammonium ions. The alumina can serve as a support for the urease. The second layer of activated carbon **1006** can capture any urease that migrates out of alumina and urease layer **1007** prior to exiting the first module **1001.** The first module **1001** can be a single use module, or can be a multiple use module with replenishment of the urease. The second module **1002** can have zirconium phosphate **1005.** After dialysis, zirconium phosphate **1005** will contain bound potassium, calcium, magnesium, and ammonium ions, which can be replaced with sodium and hydrogen ions by the recharging process described herein. Third module **1003** can contain zirconium oxide **1004.** After use, the zirconium oxide **1004** will contain bound phosphate, fluoride and other anions, which can be replaced with hydroxide anions through the recharging process described herein. The flow direction of flow of dialysate through the sorbent cartridge is shown by the arrow in FIG. 10. The recharging solutions can also flow through the reusable sorbent modules in an opposite direction to improve the efficiency of the recharging process.

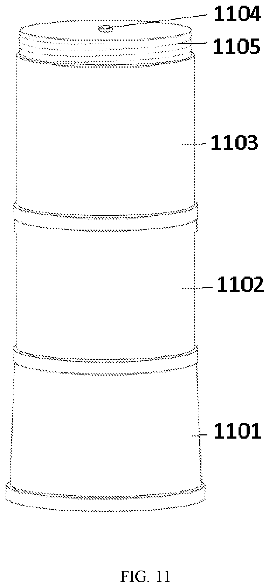

FIG. 11 illustrates another non-limiting example of a modular sorbent cartridge that can be used in the recharging process described herein. The modular sorbent cartridge can be separated into discrete modules including a first module **1101,** a second module **1102,** and a third module **1103** connected together to form a sorbent cartridge. The first module **1101** can contain activated carbon, urease, and alumina; the second module **1102** can contain zirconium phosphate; and the third module **1103** can contain zirconium oxide. One of skill in the art will understand that the modular sorbent cartridge illustrated in FIG. 11 is for illustrative purposes only, and modifications to the sorbent cartridge can be made within the scope of the invention. Alternatively, the sorbent modules can be independent with fluid lines connecting each of the sorbent modules for dialysis. During dialysis, dialysate can enter the sorbent cartridge through the bottom of first module **1101,** travel through modules **1101, 1102,** and **1103,** and exit through fluid outlet **1104.** The fluid outlet **1104** can connect to the rest of the dialysate flow path. Threaded portion **1105** on module **1103** can be used in connecting modules to each other, to the dialysate flow path, or to the recharger as described herein. The threaded portion **1105** can be included on any of the sorbent modules. Other connection types suitable for secured fluid connection in dialysis known in the art is contemplated by the invention. For example, fluid lines can be clamped directly onto fluid outlet **1104.** After dialysis, a user can disconnect the sorbent modules for disposal of single use modules and for recharging of the reusable modules.

One skilled in the art will understand that various combinations and/or modifications and variations can be made in the described systems and methods depending upon the specific needs for operation.

## Claims

1. A sorbent recharger, comprising:
at least a first receiving compartment for a first sorbent module (503); the first receiving compartment having a first sorbent module inlet and a first sorbent module outlet;
one or more fluid sources (505, 506, 507, 508) fluidly connected to the first sorbent module inlet through a first set of one or more fluid connectors;
one or more pumps (509, 510) positioned on the one or more fluid connectors for pumping fluid from the one or more fluid sources to the first sorbent module inlet; and
a first effluent line (539) fluidly connected to the first sorbent module outlet, further comprising:
at least a second receiving compartment for a second sorbent module (504); the second receiving compartment having a second sorbent module inlet and a second sorbent module outlet;
one or more fluid sources (505, 506, 507, 508) fluidly connected to the second module inlet through a second set of one or more fluid connectors; and
one or more pumps (526, 527) positioned on the one or more fluid connectors for pumping fluid from the one or more fluid sources to the second sorbent module inlet; and
a second effluent line (538) fluidly connected to the second sorbent module outlet;
wherein the first effluent line and the second effluent line are fluidly connected to a common drain line (545); and wherein the drain line is fluidly connected to any one of a drain, a common reservoir, or combinations thereof.

2. The sorbent recharger of claim 1, wherein the first sorbent module is a zirconium phosphate module; preferably wherein the one or more fluid sources is any one of a water source, a disinfectant source, and a brine source.

3. The sorbent recharger of claim 2, wherein the brine source contains any one of a solution of sodium chloride, sodium acetate, acetic acid, and combinations thereof preferably wherein the concentration of sodium chloride is between 2.5 M and 4.9 M, the concentration of sodium acetate is between 0.3 M and 1.1 M, and the concentration of acetic acid is between 0.2 M and 0.8 M.

4. The sorbent recharger of claim 1, wherein the second sorbent module is a zirconium oxide module; preferably wherein the one or more fluid sources is any one of a water source, a disinfectant source, a base source, and combinations thereof.

5. The sorbent recharger of claim 4, wherein the base source contains sodium hydroxide in a concentration of between 0.5 M and 2.0 M.

6. The sorbent recharger of any preceding claim, wherein the first sorbent module is a zirconium phosphate module; and the second sorbent module is a zirconium oxide module.

7. The sorbent recharger of any of claims 1 to 5, wherein the first sorbent module is a zirconium oxide module; and the second sorbent module is a zirconium phosphate module.

8. The sorbent recharger of claim 1, wherein the first and second sorbent modules are each zirconium phosphate modules or each zirconium oxide modules.

9. The sorbent recharger of claim 1, further comprising at least one module bypass line; wherein the module bypass line is positioned upstream of the first sorbent module inlet and is fluidly connected to the first effluent line; preferably further comprising at least a second module bypass line; wherein the second module bypass line is positioned upstream of the second sorbent module inlet and is fluidly connected to the effluent line.

10. The sorbent recharger of claim 2, wherein the drain line has a static mixer
and/or wherein either or both of:
the first module inlet is fluidly connectable to the first module outlet; and the second module inlet is fluidly connectable to the second module outlet; preferably wherein the fluid sources are selected from the group consisting of a water source, a base source, a disinfectant source, a brine source, and combinations thereof.

11. The sorbent recharger of any preceding claim, wherein the at least one fluid source is fluidly connected to a second set of one or more connectors in a second recharger; and/or wherein either or both of the sorbent module inlet and sorbent module outlet are positioned on a flexible connector.

12. A dialysis system, comprising:
one or more of the sorbent rechargers of any preceding claim, wherein the one or more sorbent rechargers are fluidly connected to a common set of the one or more fluid sources.

## Patentansprüche

1. Sorptionsmittel-Ladegerät, umfassend:
mindestens eine erste Aufnahmekammer für ein erstes Sorptionsmittelmodul (503); wobei die erste Aufnahmekammer einen ersten Sorptionsmittelmoduleinlass und einen ersten Sorptionsmittelmodulauslass aufweist;
einen oder mehrere Fluidquellen (505, 506, 507, 508), die durch einen ersten Satz von einem oder mehreren Fluidverbindern mit dem ersten Sorptionsmittelmoduleinlass in Fluidverbindung stehen;
eine oder mehrere Pumpen (509, 510), die auf dem einen oder den mehreren Fluidverbindern positioniert sind, um Fluid von der einen oder den mehreren Fluidquellen zu dem ersten Sorptionsmittelmoduleinlass zu pumpen; und
eine erste Ablaufleitung (539), die mit dem ersten Sorptionsmittelmodulauslass in Fluidverbindung steht, weiter umfassend:
mindestens eine zweite Aufnahmekammer für ein zweites Sorptionsmittelmodul (504); wobei die zweite Aufnahmekammer einen zweiten Sorptionsmittelmoduleinlass und einen zweiten Sorptionsmittelmodulauslass aufweist;
einen oder mehrere Fluidquellen (505, 506, 507, 508), die durch einen zweiten Satz von einem oder mehreren Fluidverbindern mit dem zweiten Sorptionsmittelmoduleinlass in Fluidverbindung stehen;
eine oder mehrere Pumpen (526, 527), die auf dem einen oder den mehreren Fluidverbindern positioniert sind, um Fluid von der einen oder den mehreren Fluidquellen zu dem zweiten Sorptionsmittelmoduleinlass zu pumpen; und
eine zweite Ablaufleitung (538), die mit dem zweiten Sorptionsmittelmodulauslass in Fluidverbindung steht;
wobei die erste Ablaufleitung und die zweite Ablaufleitung mit einer
gemeinsamen Abführleitung (545) in Fluidverbindung stehen;
und wobei die Abführleitung mit einem von einem Ablauf, einem gemeinsamen Reservoir oder Kombinationen davon in Fluidverbindung steht.

2. Sorptionsmittel-Ladegerät nach Anspruch 1, wobei das erste Sorptionsmittelmodul ein Zirkoniumphosphat-Modul ist; wobei vorzugsweise die eine oder die mehreren Fluidquellen eines von einer Wasserquelle, einer Desinfektionsmittelquelle und einer Solequelle ist.

3. Sorptionsmittel-Ladegerät nach Anspruch 2, wobei die Solequelle eine von einer Natriumchlorid-, Natriumacetat-, Essigsäurelösung und Kombinationen davon enthält, wobei vorzugsweise die Konzentration von Natriumchlorid zwischen 2,5 M und 4,9 M liegt, die Konzentration von Natriumacetat zwischen 0,3 M und 1,1 M liegt und die Konzentration von Essigsäure zwischen 0,2 M und 0,8 M liegt.

4. Sorptionsmittel-Ladegerät nach Anspruch 1, wobei das zweite Sorptionsmittelmodul ein Zirkoniumdioxid-Modul ist; wobei vorzugsweise die eine oder die mehreren Fluidquellen eine von einer Wasserquelle, einer Desinfektionsmittelquelle, einer Basenquelle oder Kombinationen davon ist.

5. Sorptionsmittel-Ladegerät nach Anspruch 4, wobei die Basenquelle Natriumhydroxid in einer Konzentration zwischen 0,5 M und 2,0 M enthält.

6. Sorptionsmittel-Ladegerät nach einem der vorstehenden Ansprüche, wobei das erste Sorptionsmittelmodul ein Zirkoniumphosphat-Modul ist; und das zweite Sorptionsmittelmodul ein Zirkoniumdioxid-Modul ist.

7. Sorptionsmittel-Ladegerät nach einem der Ansprüche 1 bis 5, wobei das erste Sorptionsmittelmodul ein Zirkoniumdioxid-Modul ist; und das zweite Sorptionsmittelmodul ein Zirkoniumphosphat-Modul ist.

8. Sorptionsmittel-Ladegerät nach Anspruch 1, wobei das erste und das zweite Sorptionsmittelmodul jeweils Zirkoniumphosphat-Module oder Zirkoniumdioxid-Module sind.

9. Sorptionsmittel-Ladegerät nach Anspruch 1, weiter umfassend mindestens eine Modul-Bypassleitung; wobei die Modul-Bypassleitung stromaufwärts des ersten Sorptionsmittelmoduleinlasses positioniert ist und mit der ersten Abflussleitung in Fluidverbindung steht; vorzugsweise weiter umfassend mindestens eine zweite Modul-Bypassleitung; wobei die zweite Modul-Bypassleitung stromaufwärts des zweiten Sorptionsmittelmoduleinlasses positioniert ist und mit der ersten Abflussleitung in Fluidverbindung steht.

10. Sorptionsmittel-Ladegerät nach Anspruch 2, wobei die Abführleitung einen statischen Mischer aufweist und/oder wobei eines oder beide von Folgendem vorhanden ist:
der erste Moduleinlass ist mit dem ersten Modulauslass in Fluidverbindung bringbar; und der zweite Moduleinlass ist mit dem zweiten Modulauslass in Fluidverbindung bringbar; wobei vorzugsweise die Fluidquellen ausgewählt sind aus der Gruppe, bestehend aus einer Wasserquelle, einer Basenquelle, einer Desinfektionsmittelquelle, einer Solequelle und Kombinationen davon.

11. Sorptionsmittel-Ladegerät nach einem der vorstehenden Ansprüche, wobei die mindestens eine Fluidquelle mit einem zweiten Satz von einem oder mehreren Verbindern in einem zweiten Ladegerät in Fluidverbindung steht; und/oder wobei eines oder beide von dem Sorptionsmittelmoduleinlass und dem Sorptionsmittelmodulauslass an einem flexiblen Verbinder positioniert sind.

12. Dialysesystem, umfassend:
eines oder mehrere der Sorptionsmittel-Ladegeräte nach einem der vorstehenden Ansprüche, wobei der eine oder die mehreren Sorptionsmittel- Ladegeräte mit einem gemeinsamen Satz der einen oder mehreren Fluidquellen in Fluidverbindung stehen.

## Revendications

1. Dispositif de recharge de sorbant comprenant :
au moins un premier compartiment de réception pour un premier module de sorbant (503) ; le premier compartiment de réception ayant une entrée de premier module de sorbant et une sortie de premier module de sorbant ;
une ou plusieurs sources de fluide (505, 506, 507, 508) connectées de manière fluidique à l'entrée de premier module de sorbant par l'intermédiaire d'un premier ensemble d'un ou plusieurs connecteurs de fluide ;
une ou plusieurs pompes (509, 510) positionnées sur les un ou plusieurs connecteurs de fluide pour pomper du fluide à partir des une ou plusieurs sources de fluide vers l'entrée de premier module de sorbant ; et
une première conduite d'effluent (539) connectée de manière fluidique à la sortie de premier module de sorbant, comprenant en outre :
au moins un second compartiment de réception pour un second module de sorbant (504) ;
le second compartiment de réception ayant une entrée de second module de sorbant et une sortie de second module de sorbant ;
une ou plusieurs sources de fluide (505, 506, 507, 508) connectées de manière fluidique à l'entrée de second module par l'intermédiaire d'un second ensemble d'un ou plusieurs connecteurs de fluide ; et
une ou plusieurs pompes (526, 527) positionnées sur les un ou plusieurs connecteurs de fluide pour pomper du fluide à partir des une ou plusieurs sources de fluide vers l'entrée de second module de sorbant ; et
une seconde conduite d'effluent (538) connectée de manière fluidique à la sortie de second module de sorbant ;
dans lequel la première conduite d'effluent et la seconde conduite d'effluent sont connectées de manière fluidique à une conduite de drain commune (545) ; et
dans lequel la conduite de drain est connectée de manière fluidique à l'un quelconque d'un drain, d'un réservoir commun, ou de combinaison de ceux-ci.

2. Dispositif de recharge de sorbant selon la revendication 1, dans lequel le premier module de sorbant est un module de phosphate de zirconium ; de préférence dans lequel les une ou plusieurs sources de fluide sont une quelconque parmi une source d'eau, une source de désinfectant et une source de saumure.

3. Dispositif de recharge de sorbant selon la revendication 2, dans lequel la source de saumure contient un quelconque parmi une solution de chlorure de sodium, de l'acétate de sodium, de l'acide acétique et des combinaisons de ceux-ci, de préférence dans lequel la concentration en chlorure de sodium est comprise entre 2,5 M et 4,9 M, la concentration en acétate de sodium est comprise entre 0,3 M et 1,1 M, et la concentration en acide acétique est comprise entre 0,2 M et 0,8 M.

4. Dispositif de recharge de sorbant selon la revendication 1, dans lequel le second module de sorbant est un module d'oxyde de zirconium ; de préférence dans lequel les une ou plusieurs sources de fluide sont une quelconque parmi une source d'eau, une source de désinfectant, une source de base, et des combinaisons de celles-ci.

5. Dispositif de recharge de sorbant selon la revendication 4, dans lequel la source de base contient de l'hydroxyde de sodium en une concentration comprise entre 0,5 M et 2,0 M.

6. Dispositif de recharge de sorbant selon l'une quelconque des revendications précédentes, dans lequel le premier module de sorbant est un module de phosphate de zirconium ; et le second module de sorbant est un module d'oxyde de zirconium.

7. Dispositif de recharge de sorbant selon l'une quelconque des revendications 1 à 5, dans lequel le premier module de sorbant est un module d'oxyde de zirconium ; et le second module de sorbant est un module de phosphate de zirconium.

8. Dispositif de recharge de sorbant selon la revendication 1, dans lequel les premier et second modules de sorbant sont chacun des modules de phosphate de zirconium ou chacun des modules d'oxyde de zirconium.

9. Dispositif de recharge de sorbant selon la revendication 1, comprenant en outre au moins une conduite de dérivation de module ; dans lequel la conduite de dérivation de module est positionnée en amont de l'entrée de premier module de sorbant et est connectée de manière fluidique à la première conduite d'effluent ; comprenant de préférence en outre au moins une seconde conduite de dérivation de module ; dans lequel la seconde conduite de dérivation de module est positionnée en amont de l'entrée de second module de sorbant et est connectée de manière fluidique à la conduite d'effluent.

10. Dispositif de recharge de sorbant selon la revendication 2, dans lequel la conduite de drain a un mélangeur statique et/ou dans lequel :
l'entrée du premier module peut être connectée de manière fluidique à la sortie de premier module ; et/ou l'entrée de second module peut être connectée de manière fluidique à la sortie de second module ; de préférence dans lequel les sources de fluide sont choisies dans le groupe constitué d'une source d'eau, d'une source de base, d'une source de désinfectant, d'une source de saumure, et de combinaisons de celles-ci.

11. Dispositif de recharge de sorbant selon l'une quelconque des revendications précédentes, dans lequel la au moins une source de fluide est connectée de manière fluidique à un second ensemble d'un ou plusieurs connecteurs dans un second dispositif de recharge et/ou dans lequel l'une ou l'autre de l'entrée de module de sorbant et de la sortie de module de sorbant, ou les deux, sont positionnées sur un connecteur flexible.

12. Système de dialyse comprenant :
un ou plusieurs des dispositifs de recharge de sorbant selon l'une quelconque des revendications précédentes, dans lequel les un ou plusieurs dispositifs de recharge de sorbant sont connectés de manière fluidique à un ensemble commun des unes ou plusieurs sources de fluide.
